# EUROPEAN PATENT APPLICATION

(11) **EP 4 138 089 A1**
(43) Date of publication of application: **22.02.2023**
(21) Application number: 21192426.1
(22) Date of filing: 20.08.2021
(51) Int. Cl.: G16H 20/30, A61B 5/00, A63B 21/00, A63B 24/00, G09B 19/00, A61B 5/22

(54) **METHOD, SYSTEM AND COMPUTER PROGRAM FOR PERSONALIZED TRAINING**

(71) Applicant: Deweer, N., 7500 Tournai (BE)
(72) Inventor: Deweer, Nicolas, 7500 Tournai (BE)
(74) Representative: Calysta NV

(57) **Abstract**

Method for personalizing a training exercise for a user, wherein the training exercise trains a certain muscle: the user performing a force measuring exercise, wherein the force measuring exercise infers a contraction of the same certain muscle of the user as the training exercise (S1); measuring isometrically with at least one force sensor the maximum force exerted by the user, while the user is doing the force measuring exercise (S2); personalizing the training exercise of the user based on the measured maximum force (S3).

## Description

### Technical Field

The present invention relates to a method, system and computer program for personalized training.

### Prior art

Optimized physical training depends on the objective of the training subject and its physical condition of the training subject (user). The objective of the training can be an increase in speed, force, muscle volume, endurance, etc. Training theory proposes for each objective a different kind of physical exercises to obtain the respective objective. For example, for increasing the maximal force, a high weight with a low number of repetitions is needed. For training endurance, a low weight with a high number of repetitions is required. For increasing the volume, something between the two training strategies is required. If the weight and the number of repetitions are not well chosen, the objective of the user cannot be achieved or worse, it can lead to injuries. This happens very often in fitness centres where the members train unsupervised without the proper training knowledge. Therefore, it is normally advisable to have a supervisor which regularly adapts the training to the objectives and the physical state of the user.

The biggest problem of personalized training is the correct determination of the physical state of the user which infers the correct choice of the weight for the exercise. The physical state of the user is normally determined by the maximum force of the muscle which needs to be trained. The maximum force of a muscle is normally determined by measuring the maximum number of repetitions of a contraction cycle (concentric-eccentric) of a muscle with a certain weight. This measurement is also called a repetition maximum measurement or RM, which is a maximum force measurement. Since the RM measurement itself lasts almost as long as the training exercise of the muscle itself, it would interfere with the training itself and increase the training time significantly, if such a RM measurement is done each time before doing a training exercise. This is why the determination of the RM is done maybe every six weeks and the last determined RM is then used for the time between two RM's measurements. However, the maximum force of a muscle fluctuates a lot. The maximum force depends on many factors of short-term fluctuations like the hours of sleep of the last night, the nutrition of the last days, the time after the last training of the muscle. Also long term fluctuations can play an important role like the gain in strength due to a good training. So, the RM measured with the last measurement does often not correspond to the actual RM measurement of the user, when he wants to train. Thus, even a personalized training with an expert supervisor looses often effect due to the unprecise measurement of the RM measurement of the user.

US10409961 proposes a force sensor which can be placed in a shoe or a glove to determine the weight currently carried by the user. The maximum force of the user can be determined by doing an exercise with a maximum high weight repeated for a low number of times, e.g. once or twice. The maximum weight is chosen such that the user can repeat the exercise only a few times. The maximum number of weights is then determined by the sensor by determining the maximum number of repetitions and the weight eccentrically carried by the muscle. An application connected with the sensor can then instantly calculate the optimized training for this muscle for the user based on the measured maximum force of the muscle and based on the objective of the user. When this method is used before the first exercise for the muscle to determine maximum eccentric force of the muscle and thus the optimized personal training program for the muscle, there is a sever risk of injury, because the cold muscle will be charged with at least one full eccentric movement of the muscle with the heaviest weight the user can carry.

An alternative method to measure physical state of the muscle is to measure an electric signal through the muscle during an isometric muscle exercise. This is for example suggested in US10058265, EP1859737, US10688345, US20190343459 or CN109363672. The electrical measurement can be used to prepare a personalized training of the respective muscle to obtain the best effect for the objective of the user. However, this requires the placement of electrodes for each muscle and before each exercise which is complicated. In addition, the measured electrical signal does not only depend on the physical state of the muscle, but also on the resistance of the skin, the contact of the electrode, the humidity, etc, so that these electrical measurements are not very trustworthy.

This is why still nowadays personalized training still does not really consider the physical state of the user or of its muscle for the training exercise on that day of the exercise.

### Brief summary of the invention

It is the object of the invention to provide an improved personalized training method, system and computer program.

According to the invention, this object is solved by a method, system and computer program according to the independent claims.

By measuring the maximum force isometrically, almost no time is lost for the measurement such that the measurement can be done before each exercise. This allows to personalize the training much more precise to the current state of the muscle without doing long RM measurements before the training.

The dependant claims refer to further advantageous embodiments.

The use of a training machine has the advantage that the position of the user is better defined than in a free exercise so that the measured isometric force corresponds with a higher certainty to the true maximum force of the user. In particular, when the force measuring exercise and the training exercise are done with the same weight training machine, the error of the maximum force measurement for the training exercise is very low.

Other embodiments according to the present invention are mentioned in the appended claims, the subsequent description of

### Brief description of the Drawings

Figure 1 is a schematic view of a system according to the invention.
Figure 2 is a flow chart explaining the major steps of the method of the present invention.
Figure 3 is an exemplary table for the calculation of the personalized weight for the personalized training exercise based on the maximum force of the user.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

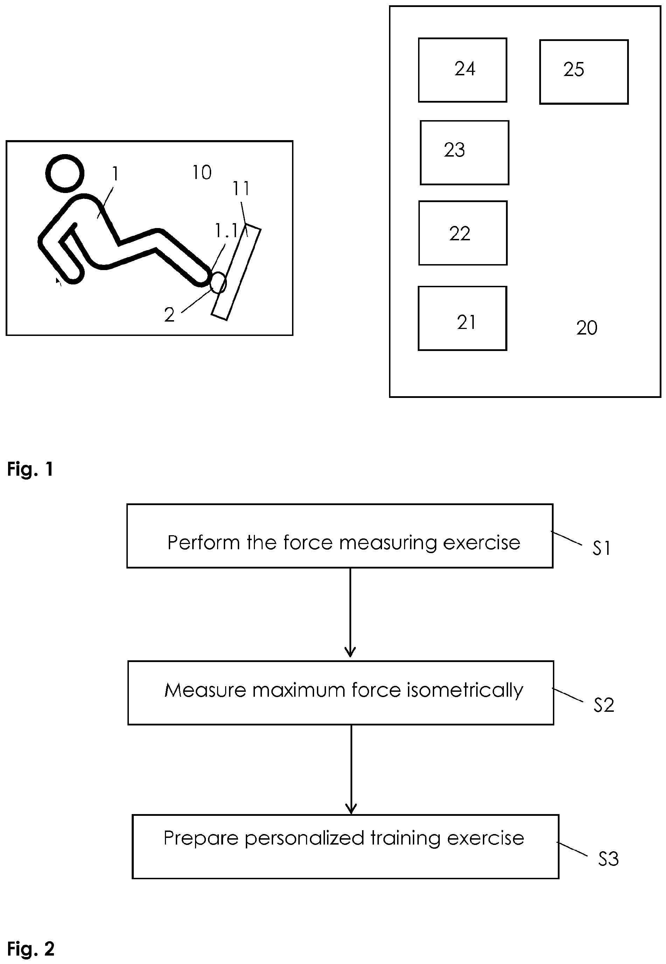

Fig. 1 shows an embodiment for the system according to the invention. The system comprises at least one pressure sensor 2 and a personalized training creator 20. Preferably, the system comprises further a weight training machine 10.

Fig. 2 shows an embodiment of a method for preparing a personalized training exercise according to the invention.

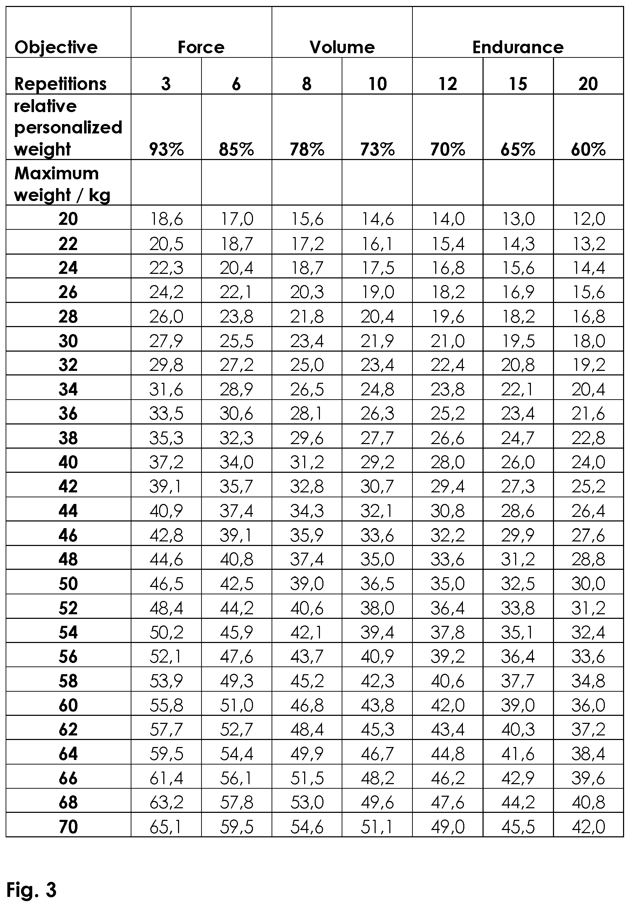

Fig. 3 shows an exemplary table how to personalize a training exercise based on the objectives and the maximum force of the user.

The invention can be further realised by a computer program.

The method, the computer program, and the system according to the invention have the purpose to provide a personalized training exercise for a user 1. Before describing the embodiments of the method, computer program and system according to the invention in more detail, some terms are defined in the following.

The user 1 is a human person for which a personalized training exercise shall be provided.

The training exercise defines at least one exercise position of the body of the user 1 which causes a contraction of a certain muscle of the user 1. If the training exercise involves a movement, the training exercise defines a sequence of exercise positions of the body of the user 1. The movement or the sequence of exercise positions is preferably periodically repeated. One period of the movement or sequence of exercise positions is called a repetition. Preferably, the training exercise comprises a defined weight to be carried by the user 1 in addition to its body weight and/or a number of repetitions. The number of repetitions can be performed with or without breaks in between. The number of repetitions can comprise a number of runs with each run comprising a number of sub-repetitions. Normally, the subnumber of repetitions of each run are repeated without break in between, while between the last repetition of one run and the first repetition of the subsequent run are normally separated by a break and/or another training exercise. For example, the training exercise can have three runs each with 10 sub-repetitions so that the training exercise has a total of 30 repetitions. The certain muscle refers to the main muscle or the main muscle group trained by the training exercise.

The training exercise is preferably realised with a weight training machine 10. However, the training exercise can also be performed with free weights or with the body weight. The personalization of the training exercise to the user refers preferably to the determination of the weight and the number of repetitions based on the physical state of the user and based on the objective of the user. However, other forms of personalization of the training exercise are possible. For example, for body weight exercises, the personalization is just the number of repetitions and for isometric exercises, the personalization would rather be the time of holding a certain isometric exercise position. The weight of the training exercise is a general measure of the additional force the user needs to perform the movement of the body of the training exercise compared to the movement just with the body weight. The additional force is often realised mechanically by physical weight objects, but could also be generated electrically, hydraulically, or pneumatically, or in other ways.

The weight training machine 10 is a training machine which allows to select the weight, i.e. the additional force compared to the body weight, to be moved by the user 1 during the training exercise. One common weight training machine is a weight-stack machine where the different weight plates are stacked, and the desired final weight can be selected by inserting a pin in the weight stack of the training machine. Also, plate-loaded machines can be used. However, hydraulic and air-pressure machines or electronic machines which create the additional force corresponding to the "weight" hydraulically, pneumatically or electrically can be used. Many other weight training machines can be used. Fig. 1 shows exemplarily a leg press as a weight training machine 10.

The weight training machine 10 has a force receiving portion 11 for receiving the force of the user 1. The force receiving portion 11 is the portion of the weight training machine 10 which receives the force from the user 1 to move the weight of the weight training machine 10 to perform the training exercise. For most weight training machines 10, the force receiving portion 11 comprises one or two handles, e.g. a handle bar to be hold by the hand(s) of the user 1, when performing the training exercise with the weight training machine 10, and/or one two foot or foot portions to receive one foot or two feet of the user 1, when performing the training exercise with the weight training machine 10. However, the force receiving portion 11 can also be something else, e.g. a sleeve to be placed at a certain point on the body of the user 1.

The weight training machine 10 allows preferably to hold or block the force receiving portion 11 in one machine exercise position. This can be realized for example by selecting the weight of the weight training machine 10 high enough that it cannot be moved by the user 1, e.g. the maximum weight. This pushes the weight training machine 10 in its initial position which then represents the one machine exercise position. However, it is also possible that the training machine 10 can be blocked in at least one of the machine exercise position.

The weight training machine 10 is preferably a guided weight training machine 10, i.e. a weight training machine 10 which guides the movement of the weight during the training exercise done with the weight training machine 10. Examples for such guided weight training machines 10 are a leg press, a chest press, a Smith machine, a cable triceps bar, a cable biceps bar to name just a few. However, it is also possible in a less preferred embodiment to use a weight training machine 10 with free weights, for example a power rack. Such a power rack which allows to define certain starting positions of exercises with weight bars shall still be considered as a weight training machine 10 which can hold the force receiving portion 11 (handlebar) in one machine exercise position, i.e. the starting position of the exercise with the handle bar.

The training exercise defines a force transmitting position 1.1 of the user 1. The force transmitting position 1.1 of the user 1 is a position on the body of the user 1 which transmits the force created by the user 1 during the training exercise outside of the body. The force transmitting position 1.1 of the user 1 is very often a foot or a hand of the user 1. However, it can also be another position on the body of the user 1, e.g. a position where a sleeve of a cable machine is fixed or the elbow for a chest press. Many training exercises comprise two or more force transmitting positions like two feet (like a leg press) or two hands (like a biceps cable bar) or the hands and the feet.

When a muscle performs an exercise, i.e. works, it contracts. There are different types of muscle contraction depending on the type of change of the muscle length. An isometric muscle contraction defines a muscle which contracts while the length of the muscle remains constant. That means the muscle holds the position of the body parts which are controlled by the muscle static, i.e. the muscle does neither shorten nor lengthen during the contraction of the muscle. An eccentric muscle contraction is a contraction of the muscle while lengthening the muscle. A concentric muscle contraction is a contraction of the muscle while shortening the muscle. During an eccentric and/or concentric muscle contraction, the length of the muscle changes during the contraction, while in an isometric muscle contraction, the length of the muscle stays constant. An isometric exercise is an exercise in which the main muscle to be trained by the exercise performs an isometric muscle contraction. An eccentric and/or concentric exercise is an exercise in which the main muscle to be trained by the exercise performs an eccentric and/or concentric muscle contraction.

The training exercise is preferably an eccentric and/or concentric exercise with a defined number of repetitions, i.e. an exercise comprising an eccentric and/or concentric exercise. The personalization would preferably define the number of repetitions and/or the weight used for the training exercise. However, the training exercise could also be an isometric exercise. In this case, the personalization could be the duration of holding the isometric exercise position and/or the weight used for the training exercise.

The invention distinguishes two exercises. First, the training exercise which shall be personalized, and second, a force measuring exercise. Both are exercises aiming to contract/train the same muscle. While the training exercise shall be personalized by the present system, method and computer program according to the invention, the force measuring exercise aims to measure the current maximum force of the user 1. Since the invention uses the maximum force measured during the force measuring exercise, the training exercise can only be performed or can only start after the force measuring exercise has ended. According to the invention, the force measuring exercise is an isometric exercise in which the user 1 performs the force measuring exercise isometrically and with maximum force. This isometric maximum force of the user 1 for the force measuring exercise is measured with a force sensor 2. The training exercise can then be personalized for the user 1 based on the maximum force of the user 1 measured for the force measuring exercise. Since the force measuring exercise is performed isometrically, a very short application of the maximum force is sufficient to measure the current maximum force of the user 1. This leads to very short measurement times for the maximum force of the user 1. This allows to perform the maximum force measurement for each training exercise just before doing the training exercise without increasing remarkably the time of the training of the user 1 by the force measurement. This allows to create a highly personalized training exercise respecting the exact maximum force of the user 1 just at the moment of the training exercise without increasing the training length or complicating much the training. Especially, this allows to consider the exhaustion of the muscles right before the training exercise and thus to personalize the training exercise precisely to the maximum force of the user 1 at the moment of performing the training exercise. In addition, the isometric application of the maximum force for a short time is much less receptive for injuries than the contraction-moving measurement of the maximum force with a heigh weight with a low number of repetitions as proposed by the state of the art.

Preferably, the training exercise is an eccentric and/or concentric exercise with a certain number of repetitions and/or with a certain weight. The force measuring exercise corresponds preferably to one exercise position of the training exercise. The force measuring exercise consists in applying in this one exercise position the maximum force. Preferably, the same weight training machine 10 is used for the training exercise and for the force measuring exercise. The one exercise position of the training position defining the isometric position of the force measuring exercise is preferably one defined position of the weight training machine, preferably its initial position.

The steps of the embodiment of a method for personalizing the training exercise according to the invention of Fig. 2 will now be described in more detail.

In a first step S1, the user 1 performs the force measuring exercise.

In a preferred embodiment, the force measuring exercise corresponds to one exercise position of the training exercise, in which the user applies/exerts isometrically its maximum force. This has the advantage that the maximum force exerted isometrically during the force measuring exercise uses the same muscles as the training exercise. However, in a less preferred embodiment, it is also possible that the force measuring exercise contracts the same muscle as the training exercise, but with another exercise position than the training exercise. However, since the muscle force depends also a bit from the exercise position, the measured maximum force is more precise, if the exercise position of the force measuring exercise corresponds to one of the exercise positions of the training exercise to be personalized.

In a preferred embodiment, the force measuring exercise is performed with a weight training machine 10. The weight training machine 10 defines the exercise position by a certain position of the force receiving portion 11 of the weight training machine 10, preferably the initial position of the weight training machine. The initial position of the weight training machine is the position in which the force receiving portion 11 is forced by the weight of the training machine. The weight training machine is configured such that the force receiving portion 11 of the weight training machine 10 is blocked, i.e. does not move. This can be achieved by selecting in the weight training machine 10 a weight which cannot be moved by the user 1, e.g. the maximum weight of the weight training machine 10. However, this can also be achieved by mechanically blocking the force receiving portion 11 of the weight training machine in the exercise position by other means. The force measuring exercise of the user 1 consists in exerting or applying (in the one exercise position) the maximum force against the blocked force receiving portion 11. Since the maximum force is exerted by the user 1 against the blocked force receiving portion 11, the (muscle of the) user 1 does not move and the user 1 exerts the maximum force isometrically, i.e. without changing the length of the contracted muscles. The user 1 applies in the force measuring exercise his/her force in the opposite direction with respect to the force applied by the weight of the weight training machine 10 on the force receiving portion 11 of the weight training machine 10. The weight training machine 10 has the advantage that the exercise position is normally well defined.

However, it is also possible to do the force measuring exercise without a weight training machine 10. Instead of pushing against a force receiving portion 11 of a weight training machine 10, the user 1 could simply push against a wall or another fixed object in the exercise position. Instead of pulling a force receiving portion 11 of a weight training machine 10, the user could pull a fixed object in the exercise position. However, the weight training machine 10 facilitates often to have the blocked receiving portion 11 in the right position and often supports the user 1 to take the right exercise position so that often the measured maximum force is more precise with a weight training machine 10.

In step S2, the maximum force exerted isometrically by the user 1 during the force measuring exercise is measured with a force sensor 2.

The force sensor 2 is configured to sense a force exerted on the sensor 2. The force sensor 2 preferably measures directly the force exerted on the sensor and/or does not require to measure an electrical activity of the muscle of the user 1 to determine the physical state, in particular the maximum force of the user 1. The force sensor 2 is preferably a pressure sensor, i.e. a force sensor 2 sensing a pressure force exerted on the force sensor 2. However, it is also possible to use a tension sensor to measure a tensile force exerted on the force sensor 2.

In a preferred embodiment, the force sensor 2, preferably a pressure sensor 2 is placed in the force transmitting position of body of the user 1. If there are two or more force transmitting positions 1.1 for the force measuring exercise, it is possible to place two or more force sensors 2 at the two or more force transmitting positions 1.1. This allows to determine the maximum force with a higher quality. However, for two symmetric force transmitting positions 1.1, e.g. the two feet 1.1 of the user 1 for the leg press 10 shown in Fig. 1, it is also possible to apply just one force sensor 2 on one foot 1.1 and assume that the same force is applied over the other foot 1.1.

The force sensor 2, preferably the pressure sensor 2 is hold at the force transmitting position 1.1 of the body with a fixing means. The fixing means is configured to hold the pressure sensor in the transmitting position 1.1 of the body with the need of the user 1 hold the pressure sensor otherwise with his hands or fingers. The fixing means can be garment like a glove, sock, shoe, shirt, pants or sleeve worn by the user. The fixing means is preferably a sleeve. The sleeve can be realized by a textile, silicon or any other suitable material. In one embodiment, the sleeve is made out of an elastic material so that the sleeve can be worn at transmitting positions 1.1 with different diameters. This allows to use the same sleeve at different transmitting positions 1.1 like foot, hand, knee, ancle, upper leg, elbow, upper arm, etc. However, it is also possible that the sleeve has a mechanism to change the diameter of the sleeve similar to the mechanisms known from a wristwatch. Preferably, the fixing means comprises a sensor recess for receiving the pressure sensor 2. The recess can be a pocket, a notch or any other recess which allows to hold the pressure sensor 2 in its place, i.e. in the recess. The pressure sensor 2 can be hold by a snap-fit, form-fit or a fixing mechanism in the sensor recess. The fixing means can comprise more than one or with a sleeve. The sleeve is preferably elastic or adjustable so that the same sleeve can be used at different body parts with different sleeve diameters, like foot, hand, knee, ancle, upper leg, elbow, upper arm, etc. Preferably, the garment or sleeve comprises a pocket in which the pressure sensor 2 can be placed. The garment or sleeve can comprise more than one pocket at different positions to place the pressure sensor 2 at different alternative positions and/or to place more than one pressure sensor 2 at two or more positions of the garment or sleeve. The force sensor 2 can for example be realized as a flat cylinder or a disc (like a coin) which is inserted in a pocket of the garment or the sleeve. However, it would also be possible to integrate the force sensor 2 in the garment or the sleeve.

If the force measuring exercise is performed with a weight training machine 10, the force sensor 2 is placed during the force measuring exercise between the force transmitting position 1.1 and the force receiving portion 11 of the weight training machine 10 such that the force exerted/applied by the user 1 can be measured with the force sensor 2. In the following some examples are described for illustration purposes without limiting the invention to any of these examples. For a weight training machine which requires the user 1 to pull a bar as force receiving portion 11 against the weight of the weight training machine 10 with the hand(s) as force transmitting position(s), the pressure sensor 2 can be placed between the pulling fingers or part of the hand and the bar, like for a biceps cable bar. For a weight training machine 10 which requires the user 1 to pull a sleeve as force receiving portion 11 against the weight of the weight training machine 10 with a part of the body of the user 1 as force transmitting position(s), the pressure sensor 2 can be placed between the portions of the sleeve and the part of the body of the user 1 over which the force is transmitted. The pressure sensor 2 could be placed with an elastic or adjustable sleeve at the respective part of the body of the user 1, i.e. the force transmitting position 1.1.

However, it is also possible that the weight training machine 10 comprises the force sensor 2. For example, some weight training machines 10 allows to change the force receiving portion 11 connected at the end of the cable. This allows to change between a bar, a sleeve and many more force receiving portions 11 of the weight training machine 10. This would for example allow to place between the end of the cable and the force receiving portion 11 a force sensor 2, preferably a tension sensor 2 to measure isometrically the maximum force of the user 1. It would also be possible that the weight training machine 10 has an integrated force sensor 2 and foresees a configuration in which the isometric maximum force can be measured with the force sensor 2. The weight training machine 10 could then be switched between a training mode in which the force receiving portion 11 is connected with the weights and a force measuring mode in which the force receiving portion 11 is connected to a force sensor 2 which measures the isometric force of the user 1. This force sensor 2 is in this case preferably a tension sensor 2.

The force sensor 2 sends preferably out a force signal representing the isometric maximum force of the user 1 measured by the force sensor 2 during the force measuring exercise. The force signal is preferably sent out wirelessly, preferably to a personalized training creator 20 or a smartphone. However, it is also possible to send the force signal over a wired connection, for example when the force sensor 2 and the personalized training creator 20 is arranged both in the training machine 10. The force sensor 2 measures preferably an analogue signal representing the force exerted on the force sensor 2 and preferably converts the analogue signal in a digital signal representing the maximum force. Preferably, the force signal sent out wirelessly is a digital signal comprising a digital information about the force sensed at the force sensor 2. The digital information can be simply the digital force over time curve measured at the force sensor 2 while the user 1 performs the force measuring exercise or one value representing the measured maximum force retrieved from the force over time curve.

The maximum force measured corresponds to the isometric maximum force of the user. In the state of the art, the maximum force of the user has been determined by the RM which rather corresponds to the concentric maximum force. Since the concentric maximum force corresponds only to 90% (conversion factor) of the isometric maximum force of the user and most of the training personalization plans are prepared for a concentric maximum force, the measured maximum force can be transformed (from an isometric maximum force) into a concentric maximum force, e.g. by multiplying the isometric maximum force with the conversion factor to obtain the concentric maximum force. Thus, preferably the isometrically measured maximum force of the user is converted into a concentric maximum force of the user.

Step S3 refers to the personalization of the training exercise based on the maximum force measured. This step is preferably performed by the personalized training creator 20 and/or the computer program described in more detail below. However, it is also possible to personalize the training exercise manually based on the maximum force measured. Fig. 3 shows an example how the personalization can be calculated for the concentric maximum force of the user. The above-mentioned conversion of the isometrical maximum force into the concentric maximum force can be made either by converting the measured isometric maximum force or by integrating the conversion factor already in the table of Fig. 3. In this embodiment, the isometrical maximum force is first converted into the concentric maximum force and the table of Fig. 3 the concentric maximum force is given. However, the contrary is equally possible to obtain the mentioned conversion. Based on the objective of the user 1, the number of repetitions and the percentage of the maximum weight of the user 1 is chosen. For example, if the user 1 has as an objective to train principally volume with more importance on force than endurance, he can do his training exercises with 8 repetitions and 78% of the maximum weight measured. The personalization of the training requires then just to multiply the measured maximum force expressed in a maximum weight with the relative personalized weight percentage value related to the objective of the user 1. If the maximum force is measured as a maximum force (not as a maximum weight), the maximum force expressed as a maximum weight can simply be obtained by dividing the measured maximum force by the gravitational acceleration. This way of personalization is very simple and allows nevertheless to highly personalize the training exercise of the user 1 to its current physical state. If the personalization is done by the user 1 itself, i.e. not by a personalized training creator 20, the user 1 could use a table like in Fig. 3 to personalize the training exercise. However, there are certainly other ways of personalizing the training exercise to the objectives of the user 1 based on his/her current maximum force. The personalization can further include other parameter like one or more of the age of the user, the weight of the user, the size of the user, the frequency of training, the time and/or type of the last training and many more.

The personalized training creator 20 will be described in more detail together with system shown in Fig. 1.

The system comprises at least one force sensor 2 and the personalized training creator 20. Preferably, the system comprises further the weight training machine 10.

The personalized training creator 20 is configured to prepare/calculate a personalization of the training exercise. The personalized training creator 20 is a computerized means. The personalized training creator 20 can be for example a mobile device, a combination of a mobile device and a server, a computerized means of the weight training machine 10 (alone or in combination with a server), a server (alone). The mobile device is preferably a smartphone or a tablet with an interface to the cellular phone network to establish an internet connection.

The personalized training creator 20 comprises a force sensor interface 21, a user input means 22, a user output means 23 and a processing means 24 and a storage means 25.

The force sensor interface 21 is configured to receive from the force sensor 2 a force signal indicative for the force measured by the force sensor 2, in particular for the maximum force exerted by the user 1 during the force measuring exercise on the force sensor 2. The force sensor interface 21 is connected with the force sensor 2 preferably via a wireless connection. The wireless connection is preferably a low energy and/or short-range connection, for example a Bluetooth (registered trademark) connection. However, it is also possible that the force sensor 2 is connected via a wired connection with the force sensor interface 21. The force sensor interface 21 preferably sends to the force sensor 2 a force measurement command indicating a force measurement time for measuring the force with the force sensor 2. Thus, the force sensor 2 receives from the force sensor interface 21 the force measurement time in which the force sensor 2 measures then the force exerted on the force sensor 2. The force measurement time could for example comprise a measurement start command to start the force measurement at the force sensor 2 and a measurement end command to end the force measurement. The force measurement time could also comprise a measurement start command or time and a duration for the force measurement. The start command is a command to start immediately the force measurement while the measurement start time would be the command to start the measurement at a certain time. Instead of receiving the duration with the force measurement time, the duration could also be predetermined in the force sensor2 and not transmitted over the force measurement time. The force measured in the force measurement time with the force sensor 2 is preferably a time series of the force during the force measurement time. The maximum force is preferably from force series during the force measurement time by further processing. The maximum force can be determined as the maximum value of the force series, as the average value of the force series, as the average value of a time window (smaller than the duration of the force measurement time) around the maximum force value or as many other processed values. The processing can be done already in the force sensor 2 or in the force receiving portion 21 or in the processing means 24 or in a mixture of two or three of those. However, it is also possible that the force sensor 2 detects directly the maximum force value so that no processing is necessary anymore. The maximum force measured/retrieved is preferably expressed in a weight, i.e. the maximum force value is divided by the gravitational acceleration to obtain the maximum force expressed as a weight. The force sensor interface 21 is preferably arranged in the mobile device which can connect to the force sensor 2, when the mobile device is in the vicinity of the force sensor 2. However, it is also possible that the force sensor interface 21 is arranged in the server and the force sensor 2 sends the force signal over an internet connection or a long-range wireless connection. It is also possible that the force sensor interface 21 is arranged in the weight training machine 10. The system can comprise more than one force sensor 2. In this case, the previous description applies analogously for each of the force sensors 2.

The user input means 22 is configured to receive a user input. A possible user input could be the objective of the training of the user 1 which is used to personalize the training of the user 1. The received objective is preferably stored in the storage means 25. The user input means 22 can be used to receive further information about the user 1 which can be used to personalized the training exercise of the user 1 and which can also be stored in the storage means 25. The user input means 22 can further be used by the user to select the training exercise he/she wants to carry out. However, the selection of the training exercise can also be performed automatically by the personalized training creator 20. The user input means 22 can also be used to receive confirmations from the user, e.g. when a certain training exercise or the force measuring exercise has been finished and/or has not correctly been carried out. In the latter case, the force measuring exercise or the training exercise can then be repeated. The user input means 22 is preferably a touch screen. The user input means 22 is preferably arranged in the mobile device or the weight training machine 10. The user input means 22 is preferably arranged in the same device as the user output means 22 and/or the force sensor interface 21.

The user output means 23 is preferably configured to output information to the user. Preferably the user output means 23 comprises a visual output and/or an audio output. The visual output is preferably a display or a projection device. The audio output is preferably a loudspeaker or an audio output interface for a loudspeaker or ear plugs. The display is preferably the touchscreen which is also used for the user input means 22. The user output means 23 is configured preferably to output instructions to the user 1 as will be described in more detail below. The user input means 22 is preferably arranged in the mobile device or the weight training machine 10. The user output means 23 is preferably arranged in the same device as the user input means 22 and/or the force sensor interface 21.

The storage means 25 stores preferably information about the user like the training objective(s) of the user 1, the measured maximum force, etc. The storage means 25 is preferably at least partly arranged in the same device as the processing means 24. For example, the storage means 25 can be fully arranged in the mobile device or distributed over the mobile device and the server or only in the server.

The processing means 24 is configured to personalize the training exercise for the user 1 based on the maximum force of the user 1 measured with the force sensor 2. The processing means 24 is configured to personalize the training exercise for the user 1 based on the force signal received from the force sensor 2 at the force sensor interface 21. The processing means 24 is preferably configured to carry out the step S3 described above.

The processing means 24 preferably determines the maximum force (preferably expressed as a maximum weight) from the force signal received at the force sensor interface 21. When the force signal already contains the maximum force, the determination of the maximum force corresponds just in reading out this information from the force signal. If the force signal contains an information about the maximum force, e.g. the force time series, the determination of the maximum force requires still some processing of the information about the maximum force read out from the force signal. This is state of the art and will therefore not be described in more detail.

The processing means 24 is preferably further configured to control the functions of the force sensor interface 21, the user input means 22 and the user output means 23 and/or the storage means 25.

The processing means 24 is preferably at least partly arranged in the same device as the force sensor interface 21, the user input means 22, the user output means 23 and/or the storage means 25. For example, the processing means 25 can be fully arranged in the mobile device or be distributed over the mobile device and the server. It is however also possible that the processing means 24 is fully arranged in the server.

The processing means 24 runs preferably a software or computer program with instructions which perform the described functions of the processing means 24, when the software or computer program is carried out by the processing means 24. Subsequently, if it is written the computer program is configured to carry out a certain function, it shall mean that the computer program comprises instructions which, when executed on the processing means 24 or the personalized training creator, would perform the certain function. Subsequently, the function of the computer program, the processing means 24 and the personalized training creator 20 are described all together in the context of the computer program. It is however clear that the same functions could be realized in the personalized training creator 20 also without a computer program.

The computer program is preferably configured to selecting a training exercise to be personalized. The training exercise can be selected by the computer program based on a training program for the user. Such a training program comprises normally a plurality of training exercises. However, it is also possible that the training exercise is selected based on a user input received via the user input means 22.

The computer program is configured to selecting a force measuring exercise based on the training exercise to be personalized. The force measuring exercise infers a contraction of the same certain muscle of the user as the training exercise to be personalized (see details for this selection above).

The computer program is configured to receiving from at least one force sensor a force signal representing a maximum force exerted by the user during the force measuring exercise. Preferably, the computer program is configured to give out via the user output means 23 instructions how the user 1 must perform the force measuring exercise. For example the instructions comprise one or more of the following instructions:
- information about the exercise position of the user in which the force measuring exercise is performed,
- information that a maximum force of the user 1 needs to be applied,
- information that the force of the user 1 is exerted isometrically,
- information about the time, when the maximum force of the user 1 needs to be applied (i.e. the force measurement time),
- information how to place the force sensor(s) 2 during the force measuring exercise,
- information about the configuration of the weight training machine 10 for the force measuring exercise.

This assures that the user 1 performs the maximum force measurement correctly and the received force signal indeed corresponds to the maximum force of the user 1.

The computer program is configured to determining a maximum force from the received force signal.

The computer program is configured to personalizing the training exercise based on the determined/measured maximum force of the user 1. This is preferably done as described in step S3. Preferably, the computer program is configured to give out information about the personalized training exercise, e.g. the number of repetitions and/or the weight to be used. However, it is also possible that the computer program configures directly the weight training machine 10 for the personalized training exercise, in selecting the weight determined by the personalization of the training exercise.

Preferably, the computer program is further configured to give out via the user output means 23 instructions how the user 1 must perform the training exercise. For example, the instructions comprise one or more of the following:
- information about the (sequence of) exercise position(s) of the user 1 in which the training exercise is performed,
- information about the number of repetitions,
- information about the weight to be selected for the training exercise
- information about the timing of the repetitions,
- information about the configuration of the weight training machine 10 for the training exercise.

The system according to the invention can be realized in many ways.

In one preferred embodiment, the personalized training creator 20 is realized in a mobile device of the user 1, normally his smartphone, preferably together with a server. The server preferably stores a profile for each user. For each user, the server stores at least the objective of the user 1. The mobile device of the user 1 is connected via an internet connection to the server to synchronize the data of the server with the data on the mobile device. The mobile device can for example run a special mobile application software for all the functions related to the personalized training creator 20 and the interactions with the server. The mobile device is used at least as force sensor interface 21, user input means 22 and user output means 23. The processing means 24 and/or the storage means 25 can be arranged in the mobile device (only), in the server (only) or in both of the mobile device and the server. This allows that the user has in its personal mobile device all information about his training program stored and has access quickly to the instructions for his personalized training exercise. It further allows to apply the current invention with traditional training equipment like traditional weight training machines which do not foresee any function to personalize a training. The force sensor 2 is preferably carried by the user, e.g. by a sleeve or a garment. However, it is also possible that the force sensor 2 is integrated in a weight training machine 10.

In another embodiment, it is possible to arrange in a weight training machine 10 the personalized training creator 20. In this embodiment, the force sensor 2 is preferably also integrated in the weight training machine 10. Thus, the user starting an exercise would need just select his training objective and then measure his maximum force with the force sensor 2 of the weight training machine 10. The personalized training creatorin the weight training machine 10 could then determine the personalized training exercise based on the objective of the user 1 and the maximum force measured. The personalized training creator 20 could communicate to the user 1 (via the user output means 23) the personalized training exercise, preferably the number of repetitions and the weight to be selected. The weight training machine 10 could automatically place the correct weight to be selected, especially for electrical weight training machines.

Many more realisations of the system according to the invention are possible. Instead of using a user-owned device, a gym could give out a dedicated device for the personalized training creator 20 which is used during the training e.g. in the gym and then passed to the next user 1. The user 1 would then need to identify himself in the personalized training creator 20 or select his objectives.

The invention was described for one training exercise. The invention can be used to personalize a training program with a plurality of training exercises. Preferably, prior to each training exercise a respective force measuring exercise is performed by the user 1, during which the maximum force exerted isometrically by the user 1 is measured. This allows to personalize each training exercise of the training program to the current maximum force of the user 1. It is however also possible to make one maximum force measurement for one muscle and then personalize two or more training exercises for this one muscle with the same measured maximum force.

It should be understood that the present invention is not limited to the described embodiments and that variations can be applied without going outside of the scope of the claims.

## Claims

1. Method for personalizing a training exercise for a user (1), wherein the training exercise trains a certain muscle, the method comprising the following steps:
the user (1) performing a force measuring exercise, wherein the force measuring exercise infers a contraction of the same certain muscle of the user as the training exercise (S1),
measuring with at least one force sensor (2) the maximum force exerted by the user (1), while the user (1) is doing the force measuring exercise (S2),
personalizing the training exercise of the user based on the measured maximum force (S3),
**characterized in that**
the force measuring exercise is an isometric exercise and the maximum force measured is the isometric maximum force exerted by the user (1) during the isometric force measuring exercise.

2. Method according to the previous claim, wherein the at least one force sensor (2) is at least one pressure sensor.

3. Method according to the previous claim, wherein the at least one pressure sensor (2) is positioned on at least one force transmitting position (1.1) of the body of the user (1), wherein the at least one force transmitting position (1.1) defines at least one position on the body of the user (1) where the force exerted by the user (1) is transmitted outside of the body while the user (1) does the force measuring exercise.

4. Method according to the previous claim, wherein the at least one force transmitting position (1.1) comprises at least one hand of the user (1) and/or at least one foot of the user (1).

5. Method according to claim 2 to 4, wherein each of the at least one pressure sensor (2) is hold by a sleeve or a garment worn by the user such that the pressure sensor measures the force caused by the user (1) at the respective one of the at least one force transmitting position (1.1), while doing the force measuring exercise.

6. Method according to the previous claim, wherein each of the at least one pressure sensor (2) is placed in a respective pocket of the sleeve or of the garment.

7. Method according to one of the previous claims, wherein the force measuring exercise and/or the training exercise is done with a weight training machine (10), wherein the weight training machine (10) allows to select the weight to be moved by the user (1), wherein the weight training machine (10) has a force receiving portion (11) for receiving the force of the user from the at least one force transmitting position (1.1), wherein the weight training machine (10) allows to hold or block the force receiving portion (11) in one machine exercise position.

8. Method according to the previous claim, wherein the force measuring exercise and the training exercise are done with the same weight training machine (10).

9. Method according to claim 7 or 8, wherein the maximum force of the user is measured isometrically by the following steps:
blocking the force receiving portion (11) of the weight training machine (10) in one machine exercise position,
the user exerts a maximum force of the user to the force receiving portion (11) of the weight training machine (10) in a defined user exercise position of the force measuring exercise,
measuring with the at least one force sensor (2) the maximum force exerted by the user (1) on the force receiving portion (11).

10. Method according to one of the previous claims, wherein the training exercise is based mainly on a concentric and/or eccentric contraction of the certain muscle, wherein the training exercise comprises a number of repetitions, wherein the training exercise is personalized by selecting the weight for the training exercise based on the maximum force measured and based on objectives of the user (1), wherein the number of repetitions is selected based on the objectives of the user (1).

11. Method according to one of the previous claims, wherein the maximum force is measured on the same day, preferably in the same hour of the training exercise before starting the training exercise so that the training exercise can be personalized based on the measured maximum force of the user on that day or in that hour.

12. Computer program for personalizing a training exercise for a user, wherein the training exercise trains a certain muscle, wherein the computer program comprising program instructions configured to perform the following steps, when executed on a personalized training creator (20):
selecting a force measuring exercise based on the training exercise to be personalized, wherein the force measuring exercise infers a contraction of the same certain muscle of the user (1) as the training exercise,
receiving from at least one force sensor (2) a force signal representing a maximum force exerted by the user (1) during the force measuring exercise,
determining a maximum force from received signal, personalizing the training exercise based on the determined maximum force, **characterized in that**
the received signal represents an isometrically exerted maximum force of the user (1) during the force measuring exercise.

13. Computer program according to the previous claim, wherein the program instructions are configured, when executed on the personalized training creator (20), to provide to the user (1) measurement instructions how to perform the force measuring exercise to measure the maximum force isometrically.

14. Computer program according to claim 9 or 10, wherein the program instructions are configured, when executed on the personalized training creator (20), to provide to the user training instructions how to perform the personalized training exercise, wherein the training instructions comprise a weight for the training exercise determined based on the maximum force measured and based on the objective of the user and comprises a number of repetitions based on the objective of the user.

15. System comprising at least one force sensor (2) and a personalized training creator (20), wherein the personalized training creator is configured to access and execute the computer program according to one of claims 12 to 14.
